(19) Europäisches Patentamt
European Patent Office
Office européen des brevets

(11) **EP 2 951 212 B1**

(12) **EUROPÄISCHE PATENTSCHRIFT**

(45) Veröffentlichungstag und Bekanntmachung des Hinweises auf die Patenterteilung:
**15.03.2017 Patentblatt 2017/11**

(21) Anmeldenummer: **14702469.9**

(22) Anmeldetag: **20.01.2014**

(51) Int Cl.:
*C08F 2/10* (2006.01)     *A61L 15/60* (2006.01)
*C08J 3/24* (2006.01)     *C08F 220/06* (2006.01)

(86) Internationale Anmeldenummer:
**PCT/EP2014/050980**

(87) Internationale Veröffentlichungsnummer:
**WO 2014/118024 (07.08.2014 Gazette 2014/32)**

(54) **VERFAHREN ZUR HERSTELLUNG WASSERABSORBIERENDER POLYMERPARTIKEL MIT HOHER QUELLGESCHWINDIGKEIT UND HOHER ZENTRIFUGENRETENTIONSKAPAZITÄT BEI GLEICHZEITIG HOHER PERMEABILITÄT DES GEQUOLLENEN GELBETTS**

METHOD FOR PRODUCING WATER-ABSORBING POLYMER PARTICLES WITH HIGH SWELLING RATE AND HIGH CENTRIFUGE RETENTION CAPACITY WITH SIMULTANEOUSLY HIGH PERMEABILITY OF THE SWOLLEN GEL BED

PROCÉDÉ DE PRODUCTION DE PARTICULES POLYMERES ABSORBANT L'EAU PRÉSENTANT UNE GRANDE RAPIDITE DE GONFLEMENT ET UNE CAPACITE DE RETENTION APRES CENTRIFUGATION ELEVEE, LE LIT DE GEL GONFLE PRESENTANT SIMULTANEMENT UNE GRANDE PERMEABILITE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **29.01.2013 EP 13152994**

(43) Veröffentlichungstag der Anmeldung:
**09.12.2015 Patentblatt 2015/50**

(73) Patentinhaber: **BASF SE**
**67056 Ludwigshafen am Rhein (DE)**

(72) Erfinder:
• **HAAG, Monica**
  **67061 Ludwigshafen (DE)**
• **KRAUSS, Roland**
  **67435 Neustadt (DE)**
• **GIEGER, Thomas**
  **67069 Ludwigshafen (DE)**
• **KLOCK, Volker**
  **67061 Ludwigshafen (DE)**
• **WEISMANTEL, Matthias**
  **63637 Jossgrund (DE)**

(74) Vertreter: **BASF IP Association**
**BASF SE**
**ZRX-C6**
**67056 Ludwigshafen (DE)**

(56) Entgegenhaltungen:
**WO-A1-2005/097313     WO-A1-2012/107432**
**WO-A1-2012/119969     WO-A1-2012/160174**
**WO-A1-2013/007819**

**Beschreibung**

Die vorliegende Erfindung betrifft ein Verfahren zur Herstellung wasserabsorbierender Polymerpartikel mit hoher Quellgeschwindigkeit (FSR) und hoher Zentrifugenretentionskapazität (CRC) bei gleichzeitig hoher Permeabilität des gequollenen Gelbetts durch Polymerisation einer wässrigen Monomerlösung in einem Polymerisationsreaktor mit mindestens zwei achsparallel rotierenden Wellen (Kneter), anschließender Extrusion bei hohen Temperaturen und thermischer Oberflächennachvernetzung.

Wasserabsorbierende Polymerpartikel werden zur Herstellung von Windeln, Tampons, Damenbinden und anderen Hygieneartikeln, aber auch als wasserzurückhaltende Mittel im landwirtschaftlichen Gartenbau verwendet. Die wasserabsorbierenden Polymerpartikel werden auch als Superabsorber bezeichnet.

Die Herstellung wasserabsorbierender Polymerpartikel wird in der Monographie "Modern Superabsorbent Polymer Technology", F.L. Buchholz und A.T. Graham, Wiley-VCH, 1998, Seiten 71 bis 103, beschrieben.

Die Eigenschaften der wasserabsorbierenden Polymerpartikel können beispielsweise über die verwendete Vernetzermenge eingestellt werden. Mit steigender Vernetzermenge sinkt die Zentrifugenretentionskapazität (CRC) und die Absorption unter einem Druck von 21,0 $g/cm^2$ (AUL0.3psi) durchläuft ein Maximum.

Zur Verbesserung der Anwendungseigenschaften, wie beispielsweise Permeabilität des gequollenen Gelbetts (SFC) in der Windel und Absorption unter einem Druck von 49,2 $g/cm^2$ (AUL0.7psi), werden wasserabsorbierende Polymerpartikel im allgemeinen oberflächennachvernetzt. Dadurch steigt der Vernetzungsgrad der Partikeloberfläche, wodurch die Absorption unter einem Druck von 49,2 $g/cm^2$ (AUL0.7psi) und die Zentrifugenretentionskapazität (CRC) zumindest teilweise entkoppelt werden können. Diese Oberflächennachvernetzung kann in wässriger Gelphase durchgeführt werden. Vorzugsweise werden aber getrocknete, gemahlene und abgesiebte Polymerpartikel (Grundpolymer) an der Oberfläche mit einem Oberflächennachvernetzer beschichtet und thermisch oberflächennachvernetzt. Dazu geeignete Vernetzer sind Verbindungen, die mit mindestens zwei Carboxylatgruppen der wasserabsorbierenden Polymerpartikel kovalente Bindungen bilden können.

Die Herstellung wasserabsorbierender Polymerpartikel in einem Polymerisationsreaktor mit mindestens zwei achsparallel rotierenden Wellen (Kneter) wird beispielsweise in WO 01/038402 A1, WO 03/022896 A1, WO 03/051415 A1, WO 2006/034806 A1, WO 2006/034853 A1 und WO 2009/115472 A1 beschrieben.

Die Extrusion der bei der Polymerisation entstehenden Polymergele wird in EP 0 497 623 A2 beschrieben.

WO 2005/097313 A1 offenbart wasserabsorbierende Polymerpartikel mit hoher Quellgeschwindigkeit (FSR).

WO 2012/107432 offenbart ein Verfahren zur Herstellung wasserabsorbierender Polymerpartikel mit hoher Anquellgeschwindigkeit durch Polymerisation einer Monomerlösung oder -Suspension, enthaltend mindestens ein ethylenisch ungesättigtes, säuregruppentragendes Monomer, das zumindest teilweise neutralisiert sein kann, mindestens einen Vernetzer, mindestens einen Initiator und mindestens einem Oberflächennachvernetzer.

WO 2012/160174 und WO 2013/007819 offenbaren ebenfalls ein Verfahren zur Herstellung wasserabsorbierender Polymerpartikel.

10 Aufgabe der vorliegenden Erfindung war die Bereitstellung eines verbesserten Verfahrens zur Herstellung wasserabsorbierender Polymerpartikel, insbesondere von wasserabsorbierenden Polymerpartikeln mit hoher Quellgeschwindigkeit (FSR) und hoher Zentrifugenretentionskapazität (CRC) bei gleichzeitig hoher Permeabilität des gequollenen Gelbetts (SFC).

Gelöst wurde die Aufgabe durch ein Verfahren zur Herstellung wasserabsorbierender Polymerpartikel durch Polymerisation einer Monomerlösung oder -suspension, enthaltend

a) ein ethylenisch ungesättigtes, säuregruppentragendes Monomer, das zumindest teilweise neutralisiert sein kann,
b) mindestens einen Vernetzer,
c) mindestens einen Initiator,
d) optional ein mit den unter a) genannten Monomeren copolymerisierbares ethylenisch ungesättigtes Monomer und
e) optional ein oder mehrere wasserlösliche Polymere,

in einem Polymerisationsreaktor mit mindestens zwei achsparallel rotierenden Wellen (Kneter), Trocknung des erhaltenen Polymergels, Mahlung des getrockneten Polymergels, Klassierung und thermischer Oberflächennachvernetzung, dadurch gekennzeichnet, dass mindestens 0,25 Gew.-% des Vernetzers b), bezogen auf das unneutralisierte Monomer a), eingesetzt wird, dass das Polymergel vor der Trocknung extrudiert wird, dass das Polymergel während der Extrusion eine Temperatur größer 80°C aufweist und dass bei der Extrusion weniger als 60 kWh/t an spezifischer mechanischer Energie eigetragen wird.

Während der Extrusion weist das Polymergel eine Temperatur von vorzugsweise größer 85°C, besonders bevorzugt größer 90°C, ganz besonders bevorzugt größer 92°C, auf. Während der Extrusion weist das Polymergel eine Temperatur von vorzugsweise weniger als 150°C, besonders bevorzugt weniger als 120°C, ganz besonders bevorzugt weniger als 105°C, auf.

Um ein Abkühlen des Polymergels während der Extrusion zu vermeiden wird der Extruder vorzugsweise begleitbeheizt, besonders bevorzugt mit Heizdampf.

Das Verhältnis von Länge zu Durchmesser des Extruders beträgt vorzugsweise weniger als 6, besonders bevorzugt weniger als 5,5, ganz besonders bevorzugt weniger als 5. Es werden also vorteilhaft kurze Extruder eingesetzt. Dadurch werden zu hohe Drücke bei der Extrusion vermieden.

Bei der Extrusion wird das Polymergel durch die Löcher einer Lochplatte gedrückt. Der Durchmesser der Löcher liegt im Bereich von vorzugsweise 2 bis 20 mm, besonders bevorzugt 5 bis 15 mm, ganz besonders bevorzugt 8 bis 12 mm.

Das Öffnungsverhältnis der Lochplatte, d.h. das Verhältnis von Summe der Fläche der Lochöffnungen zur Gesamtfläche der Lochplatte, liegt im Bereich von vorzugsweise 5 bis 50%, besonders bevorzugt 7 bis 30%, ganz besonders bevorzugt 10 bis 20%.

Die Dicke der Lochplatte, d.h. die Länge der Löcher in der Lochplatte liegt im Bereich von vorzugsweise 5 bis 50 mm, besonders bevorzugt 10 bis 40 mm, ganz besonders bevorzugt 15 bis 35 mm.

Der an der Lochplatte anstehende Druck liegt im Bereich von vorzugsweise 5 bis 50 bar, besonders bevorzugt 10 bis 40 bar, ganz besonders bevorzugt 15 bis 35 bar.

Die bei der Extrusion eingetragene spezifische mechanische Energie (SME) beträgt vorzugsweise von 2 bis 60 kWh/t, besonders bevorzugt von 5 bis 50 kWh/t, ganz besonders bevorzugt von 10 bis 40 kWh/t, und kann beispielsweise über das Verhältnis von Innenlänge zu Innendurchmesser des Extruders (L/D) beeinflusst werden. Die spezifische mechanische Energie (SME) ist die Motorleistung des Extruders in kW dividiert durch den Durchsatz an Polymergel in t/h.

In einer bevorzugten Ausführungsform der vorliegenden Erfindung wird die Polymerisation in Gegenwart eines inerten Gases und unter Überdruck durchgeführt.

Geeignete inerte Gase sind Stickstoff, Kohlendioxid, Wasserdampf und Argon. Die Polymerisationsreaktion wird durch Sauerstoff gehemmt. Daher sollte das inerte Gas vorzugsweise weniger als 0,001 Vol.-%, besonders bevorzugt weniger als 0,0005 Vol.-%, ganz besonders bevorzugt weniger als 0,0002 Vol.-%, Sauerstoff enthalten. Vorteilhaft wird er Polymerisationsrektor kontinuierlich von dem inerten Gas durchströmt. Der Inertgasvolumenstrom beträgt vorzugsweise von 0,001 bis 5 m$^3$/h pro m$^3$ Reaktorvolumen, besonders bevorzugt von 0,01 bis 2 m$^3$/h pro m$^3$ Reaktorvolumen, ganz besonders bevorzugt von 0,2 bis 1 m$^3$/h pro m$^3$ Reaktorvolumen.

Als Inertgas wird vorzugsweise Stickstoff, besonders bevorzugt in technischer Qualität, eingesetzt. Technischer Stickstoff enthält üblicherweise mindestens 99,8 Vol.-% Stickstoff und weniger als 0,0005 Vol.-% Sauerstoff.

Der Überdruck im Polymerisationsreaktor beträgt vorzugsweise von 1 bis 500 mbar, besonders bevorzugt von 5 bis 100 mbar, ganz besonders bevorzugt von 10 bis 30 mbar.

Die im erfindungsgemäßen Verfahren einsetzbaren Polymerisationsreaktoren weisen mindestens zwei achsparallel rotierende Wellen auf, wobei sich auf den Wellen üblicherweise mehrere Knet- und Transportelemente befinden.

Im erfindungsgemäßen Verfahren einsetzbare Polymerisationsreaktoren sind beispielsweise von der List AG (Arisdorf; Schweiz) erhältlich und in der CH 664 704 A5, EP 0 517 068 A1, WO 97/12666 A1, DE 21 23 956 A1, EP 0 603 525 A1, DE 195 36 944 A1 und DE 41 18 884 A1 beschrieben.

Solche Polymerisationsreaktoren mit mindestens zwei Wellen erzielen durch die Anordnung der Knet- und Transportelemente eine hohe Selbstreinigung, die für eine kontinuierliche Polymerisation eine wichtige Anforderung ist. Vorzugsweise rotieren die beiden Wellen gegenläufig zueinander.

Auf der Rührwelle sind die Scheibensegmente propellerartig angeordnet. Als Knet- und Transportelemente sind beispielsweise wandgängige Mischbarren sowie L- oder U-förmig ausgeformte Aufsätze geeignet.

Der vorliegenden Erfindung legt die Erkenntnis zugrunde, dass sich die Eigenschaften wasserabsorbierender Polymerpartikel verbessern lassen, wenn im Polymerisationsreaktor mit mindestens zwei achsparallel rotierenden Wellen hergestellte höher vernetzte Polymergele zusätzlich bei höheren Temperaturen extrudiert werden. Die dabei auftretenden Scherkräfte führen zu raueren Polymerpartikeln, die nach der thermischen Oberflächennachvernetzung eine hohe Quellgeschwindigkeit (FSR) und eine hohe Zentrifugenretentionskapazität (CRC) aufweisen. Bislang führte die Erhöhung der Quellgeschwindigkeit (FSR) immer zu einer Senkung der Zentrifugenretentionskapazität (CRC) bei vergleichbarer Permeabilität des gequollenen Gelbetts (SFC) oder zu einer Senkung der Permeabilität des gequollenen Gelbetts (SFC) bei vergleichbarer Zentrifugenretentionskapazität (CRC).

Zu hohe Energieeinträge bei der Extrusion führen allerdings zu einer Verschlechterung der Quellgeschwindigkeit (FSR) und der Zentrifugenretentionskapazität (CRC) und sind daher zu meiden.

Im Folgenden wird die Herstellung der wasserabsorbierenden Polymerpartikel näher erläutert:

Die wasserabsorbierenden Polymerpartikel werden durch Polymerisation einer Monomerlösung oder -suspension hergestellt und sind üblicherweise wasserunlöslich.

Die Monomeren a) sind vorzugsweise wasserlöslich, d.h. die Löslichkeit in Wasser bei 23°C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 35 g/100 g Wasser.

Geeignete Monomere a) sind beispielsweise ethylenisch ungesättigte Carbonsäuren, wie Acrylsäure, Methacrylsäure, und Itaconsäure. Besonders bevorzugte Monomere sind Acrylsäure und Methacrylsäure. Ganz besonders bevorzugt ist Acrylsäure.

Weitere geeignete Monomere a) sind beispielsweise ethylenisch ungesättigte Sulfonsäuren, wie Styrolsulfonsäure und 2-Acrylamido-2-methylpropansulfonsäure (AMPS).

Verunreinigungen können einen erheblichen Einfluss auf die Polymerisation haben. Daher sollten die eingesetzten Rohstoffe eine möglichst hohe Reinheit aufweisen. Es ist daher oft vorteilhaft die Monomeren a) speziell zu reinigen. Geeignete Reinigungsverfahren werden beispielsweise in der WO 02/055469 A1, der WO 03/078378 A1 und der WO 2004/035514 A1 beschrieben. Ein geeignetes Monomer a) ist beispielsweise eine gemäß WO 2004/035514 A1 gereinigte Acrylsäure mit 99,8460 Gew.-% Acrylsäure, 0,0950 Gew.-% Essigsäure, 0,0332 Gew.-% Wasser, 0,0203 Gew.-% Propionsäure, 0,0001 Gew.-% Furfurale, 0,0001 Gew.-% Maleinsäureanhydrid, 0,0003 Gew.-% Diacrylsäure und 0,0050 Gew.-% Hydrochinonmonomethylether.

Der Anteil an Acrylsäure und/oder deren Salzen an der Gesamtmenge der Monomeren a) beträgt vorzugsweise mindestens 50 mol-%, besonders bevorzugt mindestens 90 mol-%, ganz besonders bevorzugt mindestens 95 mol-%.

Die Monomere a) enthalten üblicherweise Polymerisationsinhibitoren, vorzugsweise Hydrochinonhalbether, als Lagerstabilisator.

Die Monomerlösung enthält vorzugsweise bis zu 250 Gew.-ppm, bevorzugt höchstens 130 Gew.-ppm, besonders bevorzugt höchstens 70 Gew.-ppm, bevorzugt mindestens 10 Gew.-ppm, besonders bevorzugt mindestens 30 Gew.-ppm, insbesondere um 50 Gew.-ppm, Hydrochinonhalbether, jeweils bezogen auf das unneutralisierte Monomer a). Beispielsweise kann zur Herstellung der Monomerlösung ein ethylenisch ungesättigtes, säuregruppentragendes Monomer mit einem entsprechenden Gehalt an Hydrochinonhalbether verwendet werden.

Bevorzugte Hydrochinonhalbether sind Hydrochinonmonomethylether (MEHQ) und/oder alpha-Tocopherol (Vitamin E).

Geeignete Vernetzer b) sind Verbindungen mit mindestens zwei zur Vernetzung geeigneten Gruppen. Derartige Gruppen sind beispielsweise ethylenisch ungesättigte Gruppen, die in die Polymerkette radikalisch einpolymerisiert werden können, und funktionelle Gruppen, die mit den Säuregruppen des Monomeren a) kovalente Bindungen ausbilden können. Weiterhin sind auch polyvalente Metallsalze, die mit mindestens zwei Säuregruppen des Monomeren a) koordinative Bindungen ausbilden können, als Vernetzer b) geeignet.

Vernetzer b) sind vorzugsweise Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Polyethylenglykoldiacrylat, Allylmethacrylat, Trimethylolpropantriacrylat, Triallylamin, Tetraallylammoniumchlorid, Tetraallyloxyethan, wie in EP 0 530 438 A1 beschrieben, Di- und Triacrylate, wie in EP 0 547 847 A1, EP 0 559 476 A1, EP 0 632 068 A1, WO 93/21237 A1, WO 03/104299 A1, WO 03/104300 A1, WO 03/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO 90/15830 A1 und WO 02/032962 A2 beschrieben.

Bevorzugte Vernetzer b) sind Pentaerythrittriallylether, Tetraallyloxyethan, Methylenbismethacrylamid, 15-fach ethoxyliertes Trimethylolpropantriacrylat, Polyethylenglykoldiacrylat, Trimethylolpropantriacrylat und Triallylamin.

Ganz besonders bevorzugte Vernetzer b) sind die mit Acrylsäure oder Methacrylsäure zu Di- oder Triacrylaten veresterten mehrfach ethoxylierten und/oder propoxylierten Glyzerine, wie sie beispielsweise in WO 03/104301 A1 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5-fach ethoxylierten und/oder propoxylierten Glyzerins. Am meisten bevorzugt sind die Triacrylate des 3- bis 5-fach ethoxylierten und/oder propoxylierten Glyzerins, insbesondere das Triacrylat des 3-fach ethoxylierten Glyzerins.

Die Menge an Vernetzer b) beträgt vorzugsweise 0,25 bis 1,5 Gew.-%, besonders bevorzugt 0,3 bis 1,2 Gew.-%, ganz besonders bevorzugt 0,4 bis 0,8 Gew.-%, jeweils bezogen auf unneutralisiertes Monomer a). Mit steigendem Vernetzergehalt sinkt die Zentrifugenretentionskapazität (CRC) und die Absorption unter einem Druck von 21,0 $g/cm^2$ durchläuft ein Maximum.

Als Initiatoren c) können sämtliche unter den Polymerisationsbedingungen Radikale erzeugende Verbindungen eingesetzt werden, beispielsweise thermische Initiatoren, Redox-Initiatoren, Photoinitiatoren. Geeignete Redox-Initiatoren sind Natriumperoxodisulfat/Ascorbinsäure, Wasserstoffperoxid/Ascorbinsäure, Natriumperoxodisulfat/Natriumbisulfit und Wasserstoffperoxid/Natriumbisulfit. Vorzugsweise werden Mischungen aus thermischen Initiatoren und Redox-Initiatoren eingesetzt, wie Natriumperoxodisulfat/Wasserstoffperoxid/Ascorbinsäure. Als reduzierende Komponente wird aber vorzugsweise ein Gemisch aus dem Natriumsalz der 2-Hydroxy-2-sulfinatoessigsäure, dem Dinatriumsalz der 2-Hydroxy-2-sulfonatoessigsäure und Natriumbisulfit eingesetzt. Derartige Gemische sind als Brüggolite® FF6 und Brüggolite® FF7 (Brüggemann Chemicals; Heilbronn; Deutschland) erhältlich.

Mit den ethylenisch ungesättigten, säuregruppentragenden Monomeren a) copolymerisierbare ethylenisch ungesättigte Monomere d) sind beispielsweise Acrylamid, Methacrylamid, Hydroxyethylacrylat, Hydroxyethylmethacrylat, Dimethylaminoethylmethacrylat, Dimethylaminoethyl-acrylat, Dimethylaminopropylacrylat, Diethylaminopropylacrylat, Dimethylaminoethylmethacrylat, Diethylaminoethylmethacrylat.

Als wasserlösliche Polymere e) können Polyvinylalkohol, Polyvinylpyrrolidon, Stärke, Stärkederivate, modifizierte Cellulose, wie Methylcellulose oder Hydroxyethylcellulose, Gelatine, Polyglykole oder Polyacrylsäuren, vorzugsweise Stärke, Stärkederivate und modifizierte Cellulose, eingesetzt werden.

Üblicherweise wird eine wässrige Monomerlösung verwendet. Der Wassergehalt der Monomerlösung beträgt vorzugsweise von 40 bis 75 Gew.-%, besonders bevorzugt von 45 bis 70 Gew.-%, ganz besonders bevorzugt von 50 bis 65 Gew.-%. Es ist auch möglich Monomersuspensionen, d.h. Monomerlösungen mit überschüssigem Monomer a), beispielsweise Natriumacrylat, einzusetzen. Mit steigendem Wassergehalt steigt der Energieaufwand bei der anschließenden Trocknung und mit sinkendem Wassergehalt kann die Polymerisationswärme nur noch ungenügend abgeführt werden.

Die bevorzugten Polymerisationsinhibitoren benötigen für eine optimale Wirkung gelösten Sauerstoff. Daher kann die Monomerlösung vor der Polymerisation durch Inertisierung, d.h. Durchströmen mit einem inerten Gas, vorzugsweise Stickstoff oder Kohlendioxid, von gelöstem Sauerstoff befreit werden. Vorzugsweise wird der Sauerstoffgehalt der Monomerlösung vor der Polymerisation auf weniger als 1 Gew.-ppm, besonders bevorzugt auf weniger als 0,5 Gew.-ppm, ganz besonders bevorzugt auf weniger als 0,1 Gew.-ppm, gesenkt.

Die Säuregruppen der erhaltenen Polymergele sind üblicherweise teilweise neutralisiert. Die Neutralisation wird vorzugsweise auf der Stufe der Monomeren durchgeführt. Dies geschieht üblicherweise durch Einmischung des Neutralisationsmittels als wässrige Lösung oder bevorzugt auch als Feststoff. Der Neutralisationsgrad beträgt vorzugsweise von 25 bis 85 mol-%, besonders bevorzugt von 30 bis 80 mol-%, ganz besonders bevorzugt von 40 bis 75 mol-%, wobei die üblichen Neutralisationsmittel verwendet werden können, vorzugsweise Alkalimetallhydroxide, Alkalimetalloxide, Alkalimetallkarbonate oder Alkalimetallhydrogenkarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumkarbonat oder Natriumhydrogenkarbonat sowie deren Mischungen.

Das Polymergel wird dann vorzugsweise mit einem Bandtrockner getrocknet bis der Restfeuchtegehalt vorzugsweise 0,5 bis 15 Gew.-%, besonders bevorzugt 1 bis 10 Gew.-%, ganz besonders bevorzugt 2 bis 8 Gew.-%, beträgt, wobei der Restfeuchtegehalt gemäß der von der EDANA empfohlenen Testmethode Nr. WSP 230.2-05 "Mass Loss Upon Heating" bestimmt wird.

Bei einer zu hohen Restfeuchte weist das getrocknete Polymergel eine zu niedrige Glasübergangstemperatur $T_g$ auf und ist nur schwierig weiter zu verarbeiten. Bei einer zu niedrigen Restfeuchte ist das getrocknete Polymergel zu spröde und in den anschließenden Zerkleinerungsschritten fallen unerwünscht große Mengen an Polymerpartikeln mit zu niedriger Partikelgröße ("fines") an. Der Feststoffgehalt des Gels beträgt vor der Trocknung vorzugsweise von 25 und 90 Gew.-%, besonders bevorzugt von 35 bis 70 Gew.-%, ganz besonders bevorzugt von 40 bis 60 Gew.-%. Wahlweise kann zur Trocknung aber auch ein Wirbelbetttrockner oder ein Schaufeltrockner verwendet werden.

Das getrocknete Polymergel wird hiernach gemahlen und klassiert, wobei zur Mahlung üblicherweise ein- oder mehrstufige Walzenstühle, bevorzugt zwei- oder dreistufige Walzenstühle, Stiftmühlen, Hammermühlen oder Schwingmühlen, eingesetzt werden können.

Die mittlere Partikelgröße der als Produktfraktion abgetrennten Polymerpartikel beträgt vorzugsweise mindestens 200 $\mu$m, besonders bevorzugt von 250 bis 600 $\mu$m, ganz besonders von 300 bis 500 $\mu$m. Die mittlere Partikelgröße der Produktfraktion kann mittels der von der EDANA empfohlenen Testmethode Nr. WSP 220.2-05 "Partikel Size Distribution" ermittelt werden, wobei die Massenanteile der Siebfraktionen kumuliert aufgetragen werden und die mittlere Partikelgröße graphisch bestimmt wird. Die mittlere Partikelgröße ist hierbei der Wert der Maschenweite, der sich für kumulierte 50 Gew.-% ergibt.

Der Anteil an Partikeln mit einer Partikelgröße von größer 150 $\mu$m beträgt vorzugsweise mindestens 90 Gew.-%, besonders bevorzugt mindesten 95 Gew.-%, ganz besonders bevorzugt mindestens 98 Gew.-%.

Polymerpartikel mit zu niedriger Partikelgröße senken die Permeabilität (SFC). Daher sollte der Anteil zu kleiner Polymerpartikel ("fines") niedrig sein.

Zu kleine Polymerpartikel werden daher üblicherweise abgetrennt und in das Verfahren rückgeführt. Dies geschieht vorzugsweise vor, während oder unmittelbar nach der Polymerisation, d.h. vor der Trocknung des Polymergels. Die zu kleinen Polymerpartikel können vor oder während der Rückführung mit Wasser und/oder wässrigem Tensid angefeuchtet werden.

Es ist auch möglich in späteren Verfahrensschritten zu kleine Polymerpartikel abzutrennen, beispielsweise nach der Oberflächennachvernetzung oder einem anderen Beschichtungsschritt. In diesem Fall sind die rückgeführten zu kleinen Polymerpartikel oberflächennachvernetzt bzw. anderweitig beschichtet, beispielsweise mit pyrogener Kieselsäure.

Wird zur Polymerisation ein Knetreaktor verwendet, so werden die zu kleinen Polymerpartikel vorzugsweise während des letzten Drittels der Polymerisation zugesetzt. Es ist aber auch möglich die zu kleinen Polymerpartikel in einem dem Polymerisationsreaktor nachgeschalteten Extruder in das Polymergel einzuarbeiten.

Werden die zu kleinen Polymerpartikel sehr früh zugesetzt, beispielsweise bereits zur Monomerlösung, so wird dadurch die Zentrifugenretentionskapazität (CRC) der erhaltenen wasserabsorbierenden Polymerpartikel gesenkt. Dies kann aber beispielsweise durch Anpassung der Einsatzmenge an Vernetzer b) kompensiert werden.

Der Anteil an Partikeln mit einer Partikelgröße von höchstens 850 $\mu$m, beträgt vorzugsweise mindestens 90 Gew.-%, besonders bevorzugt mindesten 95 Gew.-%, ganz besonders bevorzugt mindestens 98 Gew.-%.

Der Anteil an Partikeln mit einer Partikelgröße von höchstens 600 $\mu$m, beträgt vorzugsweise mindestens 90 Gew.-%, besonders bevorzugt mindesten 95 Gew.-%, ganz besonders bevorzugt mindestens 98 Gew.-%.

Polymerpartikel mit zu großer Partikelgröße senken die Quellgeschwindigkeit. Daher sollte der Anteil zu großer Polymerpartikel ebenfalls niedrig sein.

Zu große Polymerpartikel werden daher üblicherweise abgetrennt und in die Mahlung des getrockneten Polymergels rückgeführt.

Die Polymerpartikel können zur weiteren Verbesserung der Eigenschaften oberflächennachvernetzt werden. Geeignete Oberflächennachvernetzer sind Verbindungen, die Gruppen enthalten, die mit mindestens zwei Carboxylatgruppen der Polymerpartikel kovalente Bindungen bilden können. Geeignete Verbindungen sind beispielsweise polyfunktionelle Amine, polyfunktionelle Amidoamine, polyfunktionelle Epoxide, wie in EP 0 083 022 A2, EP 0 543 303 A1 und EP 0 937 736 A2 beschrieben, di- oder polyfunktionelle Alkohole, wie in DE 33 14 019 A1, DE 35 23 617 A1 und EP 0 450 922 A2 beschrieben, oder $\beta$-Hydroxyalkylamide, wie in DE 102 04 938 A1 und US 6,239,230 beschrieben.

Des Weiteren sind in DE 40 20 780 C1 zyklische Karbonate, in DE 198 07 502 A1 2-Oxazolidinon und dessen Derivate, wie 2-Hydroxyethyl-2-oxazolidinon, in DE 198 07 992 C1 Bis- und Poly-2-oxazolidinone, in DE 198 54 573 A1 2-Oxotetrahydro-1,3-oxazin und dessen Derivate, in DE 198 54 574 A1 N-Acyl-2-Oxazolidinone, in DE 102 04 937 A1 zyklische Harnstoffe, in DE 103 34 584 A1 bizyklische Amidoacetale, in EP 1 199 327 A2 Oxetane und zyklische Harnstoffe und in WO 03/031482 A1 Morpholin-2,3-dion und dessen Derivate als geeignete Oberflächennachvernetzer beschrieben.

Bevorzugte Oberflächennachvernetzer sind Ethylenkarbonat, Ethylenglykoldiglycidylether, Umsetzungsprodukte von Polyamiden mit Epichlorhydrin und Gemische aus Propylenglykol und 1,4-Butandiol.

Ganz besonders bevorzugte Oberflächennachvernetzer sind 2-Hydroxyethyl-2-oxazolidinon, 2-Oxazolidinon und 1,3-Propandiol.

Weiterhin können auch Oberflächennachvernetzer eingesetzt werden, die zusätzliche polymerisierbare ethylenisch ungesättigte Gruppen enthalten, wie in DE 37 13 601 A1 beschrieben

Die Menge an Oberflächennachvernetzer beträgt vorzugsweise 0,001 bis 2 Gew.-%, besonders bevorzugt 0,02 bis 1 Gew.-%, ganz besonders bevorzugt 0,05 bis 0,2 Gew.-%, jeweils bezogen auf die Polymerpartikel.

In einer bevorzugten Ausführungsform der vorliegenden Erfindung werden vor, während oder nach der Oberflächennachvernetzung zusätzlich zu den Oberflächennachvernetzern polyvalente Kationen auf die Partikeloberfläche aufgebracht.

Die im erfindungsgemäßen Verfahren einsetzbaren polyvalenten Kationen sind beispielsweise zweiwertige Kationen, wie die Kationen von Zink, Magnesium, Kalzium, Eisen und Strontium, dreiwertige Kationen, wie die Kationen von Aluminium, Eisen, Chrom, Seltenerden und Mangan, vierwertige Kationen, wie die Kationen von Titan und Zirkonium. Als Gegenion sind Hydroxid, Chlorid, Bromid, Sulfat, Hydrogensulfat, Carbonat, Hydrogencarbonat, Nitrat, Phosphat, Hydrogenphosphat, Dihydrogenphosphat und Carboxylat, wie Acetat, Citrat und Lactat, möglich. Es sind auch Salze mit unterschiedlichen Gegenionen möglich, beispielsweise basische Aluminiumsalze, wie Aluminiummonoacetat oder Aluminiummonolaktat. Aluminiumsulfat, Aluminiummonoacetat und Aluminiumlaktat sind bevorzugt. Außer Metallsalzen können auch Polyamine als polyvalente Kationen eingesetzt werden.

Die Einsatzmenge an polyvalentem Kation beträgt beispielsweise 0,001 bis 1,5 Gew.-%, vorzugsweise 0,005 bis 1 Gew.-%, besonders bevorzugt 0,01 bis 0,8 Gew.-%, jeweils bezogen auf die Polymerpartikel.

Die Oberflächennachvernetzung wird üblicherweise so durchgeführt, dass eine Lösung des Oberflächennachvernetzers auf die getrockneten Polymerpartikel aufgesprüht wird. Im Anschluss an das Aufsprühen werden die mit Oberflächennachvernetzer beschichteten Polymerpartikel oberflächennachvernetzt und getrocknet, wobei die Oberflächennachvernetzungsreaktion sowohl vor als auch während der Trocknung stattfinden kann.

Das Aufsprühen einer Lösung des Oberflächennachvernetzers wird vorzugsweise in Mischern mit bewegten Mischwerkzeugen, wie Schneckenmischer, Scheibenmischer und Schaufelmischer, durchgeführt. Besonders bevorzugt sind Horizontalmischer, wie Schaufelmischer, ganz besonders bevorzugt sind Vertikalmischer. Die Unterscheidung in Horizontalmischer und Vertikalmischer erfolgt über die Lagerung der Mischwelle, d.h. Horizontalmischer haben eine horizontal gelagerte Mischwelle und Vertikalmischer haben eine vertikal gelagerte Mischwelle. Geeignete Mischer sind beispielsweise Horizontale Pflugschar® Mischer (Gebr. Lödige Maschinenbau GmbH; Paderborn; Deutschland), Vrieco-Nauta Continuous Mixer (Hosokawa Micron BV; Doetinchem; Niederlande), Processall Mixmill Mixer (Processall Incorporated; Cincinnati; USA) und Schugi Flexomix® (Hosokawa Micron BV; Doetinchem; Niederlande). Es ist aber auch möglich die Oberflächennachvernetzerlösung in einem Wirbelbett aufzusprühen.

Die Oberflächennachvernetzer werden typischerweise als wässrige Lösung eingesetzt. Über den Gehalt an nichtwässrigem Lösungsmittel bzw. Gesamtlösungsmittelmenge kann die Eindringtiefe des Oberflächennachvernetzers in die Polymerpartikel eingestellt werden.

Wird ausschließlich Wasser als Lösungsmittel verwendet, so wird vorteilhaft ein Tensid zugesetzt. Dadurch wird das Benetzungsverhalten verbessert und die Verklumpungsneigung vermindert. Vorzugsweise werden aber Lösungsmittelgemische eingesetzt, beispielsweise Isopropanol/Wasser, 1,3-Propandiol/Wasser und Propylenglykol/Wasser, wobei das Mischungsmassenverhältnis vorzugsweise von 20:80 bis 40:60 beträgt.

Die Oberflächennachvernetzung wird vorzugsweise in Kontakttrocknern, besonders bevorzugt Schaufeltrocknern, ganz besonders bevorzugt Scheibentrocknern, durchgeführt. Geeignete Trockner sind beispielsweise Hosokawa Bepex® Horizontal Paddle Dryer (Hosokawa Micron GmbH; Leingarten; Deutschland), Hosokawa Bepex® Disc Dryer (Hosokawa Micron GmbH; Leingarten; Deutschland), Holo-Flite® dryers (Metso Minerals Industries Inc.; Danville; USA) und Nara Paddle Dryer (NARA Machinery Europe; Frechen; Deutschland). Überdies können auch Wirbelschichttrockner eingesetzt werden.

Die Oberflächennachvernetzung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Besonders vorteilhaft wird in einem Wirbelschichttrockner gemischt und getrocknet.

Bevorzugte Reaktionstemperaturen liegen im Bereich 100 bis 250°C, bevorzugt 120 bis 220°C, besonders bevorzugt 130 bis 210°C, ganz besonders bevorzugt 150 bis 200°C. Die bevorzugte Verweilzeit bei dieser Temperatur beträgt vorzugsweise mindestens 10 Minuten, besonders bevorzugt mindestens 20 Minuten, ganz besonders bevorzugt mindestens 30 Minuten, und üblicherweise höchstens 60 Minuten.

In einer bevorzugten Ausführungsform der vorliegenden Erfindung werden die wasserabsorbierenden Polymerpartikel nach der Oberflächennachvernetzung gekühlt. Die Kühlung wird vorzugsweise in Kontaktkühlern, besonders bevorzugt Schaufelkühlern, ganz besonders bevorzugt Scheibenkühlern, durchgeführt. Geeignete Kühlersind beispielsweise Hosokawa Bepex® Horizontal Paddle Cooler (Hosokawa Micron GmbH; Leingarten; Deutschland), Hosokawa Bepex® Disc Cooler (Hosokawa Micron GmbH; Leingarten; Deutschland), Holo-Flite® coolers (Metso Minerals Industries Inc.; Danville; USA) und Nara Paddle Cooler (NARA Machinery Europe; Frechen; Deutschland). Überdies können auch Wirbelschichtkühler eingesetzt werden.

Im Kühler werden die wasserabsorbierenden Polymerpartikel auf 20 bis 150°C, vorzugsweise 30 bis 120°C, besonders bevorzugt 40 bis 100°C, ganz besonders bevorzugt 50 bis 80°C, abgekühlt.

Anschließend können die oberflächennachvernetzten Polymerpartikel erneut klassiert werden, wobei zu kleine und/oder zu große Polymerpartikel abgetrennt und in das Verfahren rückgeführt werden.

Die oberflächennachvernetzten Polymerpartikel können zur weiteren Verbesserung der Eigenschaften beschichtet oder nachbefeuchtet werden.

Die Nachbefeuchtung wird vorzugsweise bei 30 bis 80°C, besonders bevorzugt bei 35 bis 70°C, ganz besonders bevorzugt bei 40 bis 60°C, durchgeführt. Bei zu niedrigen Temperaturen neigen die wasserabsorbierenden Polymerpartikel zum Verklumpen und bei höheren Temperaturen verdampft bereits merklich Wasser. Die zur Nachbefeuchtung eingesetzte Wassermenge beträgt vorzugsweise von 1 bis 10 Gew.-%, besonders bevorzugt von 2 bis 8 Gew.-%, ganz besonders bevorzugt von 3 bis 5 Gew.-%. Durch die Nachbefeuchtung wird die mechanische Stabilität der Polymerpartikel erhöht und deren Neigung zur statischen Aufladung vermindert. Vorteilhaft wird die Nachbefeuchtung im Kühler nach der thermischen Trocknung durchgeführt.

Geeignete Beschichtungen zur Verbesserung der Quellgeschwindigkeit sowie der Permeabilität (SFC) sind beispielsweise anorganische inerte Substanzen, wie wasserunlösliche Metallsalze, organische Polymere, kationische Polymere sowie zwei- oder mehrwertige Metallkationen. Geeignete Beschichtungen zur Staubbindung sind beispielsweise Polyole. Geeignete Beschichtungen gegen die unerwünschte Verbackungsneigung der Polymerpartikel sind beispielsweise pyrogene Kieselsäure, wie Aerosil® 200, und Tenside, wie Span@ 20.

Ein weiterer Gegenstand der vorliegenden Erfindung sind wasserabsorbierende Polymerpartikel, erhältlich durch Polymerisation einer Monomerlösung oder-suspension, enthaltend

a) ein ethylenisch ungesättigtes, säuregruppentragendes Monomer, das zumindest teilweise neutralisiert sein kann,
b) mindestens einen Vernetzer,
c) mindestens einen Initiator,
d) optional ein mit den unter a) genannten Monomeren copolymerisierbares ethylenisch ungesättigtes Monomer und
e) optional ein oder mehrere wasserlösliche Polymere,

wobei mindestens 0,25 Gew.-% des Vernetzers b), bezogen auf das unneutralisierte Monomer a), eingesetzt wurde, und wobei das erhaltene Polymergel extrudiert wurde, die wasserabsorbierenden Polymerpartikel thermisch oberflächennachvernetzt wurden, die wasserabsorbierenden Polymerpartikel eine Quellgeschwindigkeit von mindestens 0,25 g/g s, eine Zentrifugenretentionskapazität von mindestens 25 g/g, eine Absorption unter einem Druck von 49,2 g/cm² von mindestens 24 g/g und eine Flüssigkeitsweiterleitung von mindestens 100 x 10⁻⁷ cm³s/g aufweisen.

Die Menge an Vernetzer b) beträgt vorzugsweise 0,25 bis 1,5 Gew.-%, besonders bevorzugt 0,3 bis 1,2 Gew.-%, ganz besonders bevorzugt 0,4 bis 0,8 Gew.-%, jeweils bezogen auf unneutralisiertes Monomer a).

Die erfindungsgemäßen wasserabsorbierenden Polymerpartikel weisen eine Quellgeschwindigkeit (FSR) von typischerweise mindestens 0,25 g/g s, vorzugsweise mindestens 0,27 g/g s, besonders bevorzugt 0,29 g/g s, ganz besonders bevorzugt 0,3 g/g s, auf. Die Quellgeschwindigkeit (FSR) der wasserabsorbierenden Polymerpartikel beträgt üblicherweise weniger als 1,0 g/g s.

Die erfindungsgemäßen wasserabsorbierenden Polymerpartikel weisen eine Zentrifugenretentionskapazität (CRC) von mindestens 25 g/g, auf. Die Zentrifugenretentionskapazität (CRC) der wasserabsorbierenden Polymerpartikel beträgt üblicherweise weniger als 60 g/g. Die Zentrifugenretentionskapazität (CRC) wird gemäß der von der EDANA empfohlenen Testmethode Nr. WSP 241.2-05 "Fluid Retention Capacity in Saline, After Centrifugation" bestimmt.

Die erfindungsgemäßen Verfahren wasserabsorbierenden Polymerpartikel weisen eine Absorption unter einem Druck von 49,2 g/cm² (AUL0.7psi) von mindestens 24 g/g, auf. Die Absorption unter einem Druck von 49,2 g/cm² (AUL0.7psi) der wasserabsorbierenden Polymerpartikel beträgt üblicherweise weniger als 35 g/g. Die Absorption unter einem Druck von 49,2 g/cm² (AUL0.7psi) wird analog der von der EDANA empfohlenen Testmethode Nr. WSP 242.2-05 "Absorption Under Pressure, Gravimetric Determination" bestimmt, wobei statt eines Drucks von 21,0 g/cm² ein Druck von 49,2 g/cm² eingestellt wird.

Die erfindungsgemäßen wasserabsorbierenden Polymerpartikel weisen eine Flüssigkeitsweiterleitung (SFC) von typischerweise mindestens 100 x 10⁻⁷ cm³s/g, vorzugsweise mindestens 110 x 10⁻⁷ cm³s/g, besonders bevorzugt 120 x 10⁻⁷ cm³s/g, ganz besonders bevorzugt 130 x 10⁻⁷ cm³s/g, auf. Die Flüssigkeitsweiterleitung (SFC) der wasserabsorbierenden Polymerpartikel beträgt üblicherweise weniger als 500 x 10⁻⁷ cm³s/g.

Die erfindungsgemäßen wasserabsorbierenden Polymerpartikel haben einen Anteil an Partikeln mit einer Partikelgröße von 300 bis 600 µm von vorzugsweise mindestens 30 Gew.-%, besonders bevorzugt mindesten 40 Gew.-%, ganz besonders bevorzugt mindestens 50 Gew.-%.

Ein weiterer Gegenstand der vorliegenden Erfindung sind Hygieneartikel, enthaltend erfindungsgemäße wasserabsorbierende Polymerpartikel.

Die Hygieneartikel enthalten üblicherweise eine wasserundurchlässige Rückseite, eine wasserdurchlässige Oberseite und dazwischen einen absorbierenden Kern aus den erfindungsgemäßen wasserabsorbierenden Polymerpartikeln und Fasern, vorzugsweise Cellulose. Der Anteil der erfindungsgemäßen wasserabsorbierenden Polymerpartikel im absorbierenden Kern beträgt vorzugsweise 20 bis 100 Gew.-%, bevorzugt 50 bis 100 Gew.-%.

Methoden:

Die nachfolgend beschriebenen, mit "WSP" bezeichneten Standard-Testmethoden werden beschrieben in: "Standard Test Methods for the Nonwovens Industry", Ausgabe 2005, gemeinsam herausgegeben von den "Worldwide Strategie Partners" EDANA (Avenue Eugene Plasky, 157, 1030 Brüssel, Belgien, www.edana.org) und INDA (1100 Crescent Green, Suite 115, Cary, North Carolina 27518, U.S.A., www.inda.org). Diese Veröffentlichung ist sowohl von EDANA als auch von INDA erhältlich.

Die Messungen sollten, wenn nicht anders angegeben, bei einer Umgebungstemperatur von 23 ± 2 °C und einer relativen Luftfeuchte von 50 ± 10 % durchgeführt werden. Die wasserabsorbierenden Polymerpartikel werden vor der Messung gut durchmischt.

Zentrifugenretentionskapazität (Centrifuge Retention Capacity)

Die Zentrifugenretentionskapazität (CRC) wird gemäß der von der EDANA empfohlenen Testmethode Nr. WSP 241.2-05 "Fluid Retention Capacity in Saline, After Centrifugation" bestimmt.

Absorption unter einem Druck von 21,0 g/cm² (Absorption under Load)

Die Absorption unter einem Druck von 21,0 g/cm² (AUL0.3psi) wird gemäß der von der EDANA empfohlenen Testmethode Nr. WSP 242.2-05 "Absorption Under Pressure, Gravimetric Determination" bestimmt.

Absorption unter einem Druck von 49,2 g/cm² (Absorption under Load)

Die Absorption unter einem Druck von 49,2 g/cm² (AUL0.7psi) wird analog der von der EDANA empfohlenen Testmethode Nr. WSP 242.2-05 "Absorption Under Pressure, Gravimetric Determination" bestimmt, wobei statt eines Drucks von 21,0 g/cm² (AUL0.3psi) ein Druck von 49,2 g/cm² (AUL0.7psi) eingestellt wird.

Quellgeschwindigkeit (Free Swell Rate)

Zur Bestimmung der Quellgeschwindigkeit (FSR) werden 1,00 g (= $W_1$) der wasserabsorbierenden Polymerpartikel in ein 25 ml Becherglas eingewogen und gleichmäßig auf dessen Boden verteilt. Dann werden 20 ml einer 0,9 gew.-%igen Kochsalzlösung mittels eines Dispensers in ein zweites Becherglas dosiert und der Inhalt dieses Glases wird dem ersten zügig hinzugefügt und eine Stoppuhr gestartet. Sobald der letzte Tropfen Salzlösung absorbiert wird, was man am Verschwinden der Reflexion auf der Flüssigkeitsoberfläche erkennt, wird die Stoppuhr angehalten. Die genaue Flüssigkeitsmenge, die aus dem zweiten Becherglas ausgegossen und durch das Polymer im ersten Becherglas absorbiert wurde, wird durch Rückwägung des zweiten Becherglases genau bestimmt (= $W_2$). Die für die Absorption benötigte Zeitspanne, die mit der Stoppuhr gemessen wurde, wird als t bezeichnet. Das Verschwinden des letzten Flüssigkeitstropfens auf der Oberfläche wird als Zeitpunkt t bestimmt.

Daraus errechnet sich die Quellgeschwindigkeit (FSR) wie folgt:

$$FSR \ [g/g \ s] = W_2/(W_1 \ x \ t)$$

Wenn der Feuchtegehalt der wasserabsorbierenden Polymerpartikel jedoch mehr als 3 Gew.-% beträgt, so ist das Gewicht $W_1$ um diesen Feuchtegehalt zu korrigieren.

Für die Bestimmung der Quellgeschwindigkeit (FSR) des Grundpolymers wird nur die Partikelgrößenfraktion von 300 bis 400 $\mu$m eingesetzt.

Flüssigkeitsweiterleitung (Saline Flow Conductivity)

Die Flüssigkeitsweiterleitung (SFC) einer gequollenen Gelschicht unter Druckbelastung von 0,3 psi (2070 Pa) wird, wie in EP 0 640 330 A1 beschrieben, als Gel-Layer-Permeability einer gequollenen Gelschicht aus wasserabsorbierenden Polymerpartikeln bestimmt, wobei die in zuvor genannter Patentanmeldung auf Seite 19 und in Figur 8 beschriebene Apparatur dahingehend modifiziert wurde, dass die Glasfritte (40) nicht mehr verwendet wird, der Stempel (39) aus gleichem Kunststoffmaterial besteht wie der Zylinder (37) und jetzt über die gesamte Auflagefläche gleichmäßig verteilt 21 gleichgroße Bohrungen enthält. Die Vorgehensweise sowie Auswertung der Messung bleibt unverändert gegenüber EP 0 640 330 A1. Der Durchfluss wird automatisch erfasst.

Die Flüssigkeitsweiterleitung (SFC) wird wie folgt berechnet:

$$SFC \ [cm^3s/g] = (Fg(t=0) \ x \ L_0)/(d \ x \ A \ x \ WP),$$

wobei Fg(t=0) der Durchfluss an NaCl-Lösung in g/s ist, der anhand einer linearen Regressionsanalyse der Daten Fg(t) der Durchflussbestimmungen durch Extrapolation gegen t=0 erhalten wird, $L_0$ die Dicke der Gelschicht in cm, d die Dichte der NaCl-Lösung in g/cm$^3$, A die Fläche der Gelschicht in cm$^2$ und WP der hydrostatische Druck über der Gelschicht in dyn/cm$^2$.

Beispiele

Beispiel 1

Durch kontinuierliches Mischen von Wasser, 50 gew.-%iger Natronlauge und Acrylsäure wurde eine 42,7 gew.-%ige Acrylsäure/Natriumacrylatlösung hergestellt, so dass der Neutralisationsgrad 69,0 mol-% betrug. Die Monomerlösung wurde nach dem Mischen der Komponenten durch einen Wärmetauscher kontinuierlich auf eine Temperatur von 30°C abgekühlt und mit Stickstoff entgast. Als mehrfach ethylenisch ungesättigter Vernetzer wurde 3-fach ethoxiliertes Glyzeryintriacrylat (ca. 85gew.-%ig) verwendet. Die Einsatzmenge, bezogen auf die eingesetzte Acrylsäure (baAS), betrug 0,35 Gew.-%. Zur Initiierung der radikalischen Polymerisation wurden folgende Komponenten eingesetzt: 0,0008 Gew.-% baAS Wasserstoffperoxid, zudosiert als 2,5gew.-%ige wässrige Lösung, 0,13 Gew.-% baAS Natriumperoxodisulfat, zudosiert als 15gew.-%ige wässrige Lösung, sowie 0,0023 Gew.-% baAS Ascorbinsäure, zudosiert als 0,5gew.-%ige wässrige Lösung. Der Durchsatz der Monomerlösung betrug 800 kg/h.

Die einzelnen Komponenten wurden kontinuierlich in einen Reaktor vom Typ List ORP 250 Contikneter (List AG, Arisdorf, Schweiz) eindosiert. Im ersten Drittel des Reaktors wurden zusätzlich 26,3 kg/h abgetrenntes Unterkorn mit einer Partikelgröße kleiner 150 $\mu$m zugesetzt. Die Reaktionslösung hatte am Zulauf eine Temperatur von 30°C. Die Verweilzeit der Reaktionsmischung im Reaktor betrug ca. 15 Minuten.

Ein Teil des so erhaltenen Polymergels wurde mit einem Extruder vom Typ SLRE 75 R (Sela Maschinen GmbH; Harbke; Deutschland) extrudiert. Die Temperatur des Polymergels bei der Extrusion betrug 95°C. Die Lochplatte hatte 12 Löcher mit einem Lochdurchmesser von 8 mm. Die Dicke der Lochplatte betrug 16 mm. Das Verhältnis von Innenlänge zu Innendurchmesser des Extruders (L/D) betrug 4. Die spezifische mechanische Energie (SME) der Extrusion betrug 26 kWh/t. Das extrudierte Polymergel wurde auf Blechen verteilt und 90 Min. bei 175°C im Umlufttrockenschrank getrocknet. Die Beladung der Bleche mit Polymergel betrug 0,81 g/cm².

Das getrocknete Polymergel wurde mittels eines einstufigen Walzenstuhles gemahlen (drei Mahlgänge, 1. Mahlgang mit Spaltbreite 1000μm, 2. Mahlgang mit Spaltbreite 600μm und 3. Mahlgang mit Spaltbreite 400μm). Das gemahlene getrocknete Polymergel wurde klassiert und eine synthetische Partikelgrößenverteilung (PSD) mit folgender Zusammensetzung abgemischt:

600 bis 710μm: 10,6 Gew.%
500 bis 600μm: 27,9 Gew.%
300 bis 500μm: 42,7 Gew.%
200 bis 300μm: 13,8 Gew.%
150 bis 200μm: 5,0 Gew.%

Das so erhaltene Grundpolymer A wurde analysiert. Die Ergebnisse sind in Tabelle 1 eingetragen.

Beispiel 2

1,2 kg Grundpolymer A aus Beispiel 1 wurde in einem Pflugschar-Mischer vom Typ M5 mit Heizmantel (Gebr. Lödige Maschinenbau GmbH; Paderborn, Deutschland) bei 23°C und einer Wellendrehzahl von 200 Umdrehungen pro Minute mittels einer Zweistoff-Sprühdüse mit 54,6 g eines Gemisches aus 0,07 Gew.-% N-Hydroxyethyl-2-oxazolidinon, 0,07 Gew.-% 1,3-Propandiol, 0,7 Gew.-% Propylenglykol, 2,27 Gew.-% einer 22gew.-%igen wässrigen Aluminiumlaktatlösung, 0,448 Gew.-% einer 0,9gew.-%igen wässrigen Sorbitanmonolauratlösung und 0,992 Gew.-% Isopropanol, die Gew.-% jeweils bezogen auf das Grundpolymer A, beschichtet.

Nach dem Aufsprühen wurde die Produkttemperatur auf 185°C erhöht und das Reaktionsgemisch 35 Minuten lang bei dieser Temperatur und einer Wellendrehzahl von 50 Umdrehungen pro Minute gehalten. Das erhaltene Produkt wurde auf Umgebungstemperatur abgekühlt und erneut mit einem 710μm Sieb klassiert. Die Fraktion mit einer Partikelgröße von kleiner 710 μm wurde analysiert. Die Ergebnisse sind in Tabelle 2 eingetragen.

Beispiel 3 (Vergleichsbeispiel)

Es wurde verfahren wie unter Beispiel 1, wobei das erhaltene Polymergel nicht extrudiert wurde. Das so erhaltene Grundpolymer B wurde analysiert. Die Ergebnisse sind in Tabelle 1 eingetragen.

Beispiel 4 (Vergleichsbeispiel)

Das Grundpolymer B aus Beispiel 3 wurde wie in Beispiel 2 thermisch oberflächennachvernetzt. Die Fraktion mit einer Partikelgröße von kleiner 710 μm wurde analysiert. Die Ergebnisse sind in Tabelle 2 eingetragen.

Beispiel 5

Es wurde verfahren wie unter Beispiel 1, wobei die Temperatur des Polymergels bei der Extrusion 85° betrug. Das so erhaltene Grundpolymer C wurde analysiert. Die Ergebnisse sind in Tabelle 1 eingetragen.

Beispiel 6

Das Grundpolymer C aus Beispiel 5 wurde wie in Beispiel 2 thermisch oberflächennachvernetzt. Die Fraktion mit einer Partikelgröße von kleiner 710 μm wurde analysiert. Die Ergebnisse sind in Tabelle 2 eingetragen.

Beispiel 7 (Vergleichsbeispiel)

Es wurde verfahren wie unter Beispiel 1, wobei die Temperatur des Polymergels bei der Extrusion 62° betrug. Das so erhaltene Grundpolymer D wurde analysiert. Die Ergebnisse sind in Tabelle 1 eingetragen.

Beispiel 8 (Vergleichsbeispiel)

Das Grundpolymer D aus Beispiel 7 wurde wie in Beispiel 2 thermisch oberflächennachvernetzt. Die Fraktion mit einer Partikelgröße von kleiner 710 μm wurde analysiert. Die Ergebnisse sind in Tabelle 2 eingetragen.

Tab. 1: Polymergel-Temperatureinfluss bei der Extrusion auf das Grundpolymer

| Bsp. | Temp. Polymergel [°C] | CRC [g/g] | AUL0.3psi [g/g] | FSR [g/gs] |
|------|----------------------|-----------|-----------------|------------|
| 3*) | --- | 37,7 | 11,0 | 0,31 |
| 1 | 95 | 34,4 | 16,4 | 0,38 |
| 5 | 85 | 34,2 | 17,0 | 0,37 |
| 7*) | 62 | 37,2 | 10,3 | 0,25 |
| *) Vergleichsbeispiele | | | | |

Tab. 2: Polymergel-Temperatureinfluss bei der Extrusion auf das Endprodukt

| Bsp. | Temp. Polymergel [°C] | SFC [$10^{-7}$ x $cm^3$s/g] | CRC [g/g] | AUL0.7psi [g/g] | FSR [g/gs] |
|------|----------------------|-----------------------------|-----------|-----------------|------------|
| 4*) | --- | 76 | 27,7 | 25,1 | 0,17 |
| 2 | 95 | 103 | 27,2 | 25,1 | 0,34 |
| 6 | 85 | 98 | 26,7 | 24,9 | 0,29 |
| 8*) | 62 | 95 | 27,0 | 23,8 | 0,22 |
| *) Vergleichsbeispiele | | | | | |

Die Beispiele 1 bis 8 zeigen, dass die Quellgeschwindigkeit (FSR) nach der Oberflächennachvernetzung mit der Polymergel-Temperatur während der Extrusion steigt.

Beispiel 9 (Vergleichsbeispiel)

Durch kontinuierliches Mischen von Wasser, 50 gew.-%iger Natronlauge und Acrylsäure wurde eine 42,7 gew.-%ige Acrylsäure/Natriumacrylatlösung hergestellt, so dass der Neutralisationsgrad 69,0 mol-% betrug. Die Monomerlösung wurde nach dem Mischen der Komponenten durch einen Wärmetauscher kontinuierlich auf eine Temperatur von 30°C abgekühlt und mit Stickstoff entgast. Als mehrfach ethylenisch ungesättigter Vernetzer wurde 3-fach ethoxiliertes Glyzeryintriacrylat (ca. 85gew.-%ig) verwendet. Die Einsatzmenge, bezogen auf die eingesetzte Acrylsäure, betrug 0,20 Gew.-%. Zur Initiierung der radikalischen Polymerisation wurden folgende Komponenten eingesetzt: 0,002 Gew.-% baAS Wasserstoffperoxid, zudosiert als 2,5gew.-%ige wässrige Lösung, 0,1 Gew.% baAS Natriumperoxodisulfat, zudosiert als 15gew.-%ige wässrige Lösung, sowie 0,01 Gew.-% baAS Ascorbinsäure, zudosiert als 0,5gew.-%ige wässrige Lösung. Der Durchsatz der Monomerlösung betrug 40 kg/h.

Die einzelnen Komponenten wurden kontinuierlich in einen Reaktor vom Typ List ORP 10 Contikneter (List AG, Arisdorf, Schweiz) eindosiert.

Die Reaktionslösung hatte am Zulauf eine Temperatur von 30°C. Die Verweilzeit der Reaktionsmischung im Reaktor betrug ca. 15 Minuten.

Ein Teil des so erhaltenen Polymergels wurde mit einem Extruder vom Typ SLRE 75 R (Sela Maschinen GmbH; Harbke; Deutschland) extrudiert. Die Temperatur des Polymergels bei der Extrusion betrug 85°. Die Lochplatte hatte 12 Löcher mit einem Lochdurchmesser von 8 mm. Die Dicke der Lochplatte betrug 16 mm. Das Verhältnis von Innenlänge zu Innendurchmesser des Extruders (L/D) betrug 4. Die spezifische mechanische Energie (SME) der Extrusion betrug 26 kWh/t. Das extrudierte Polymergel wurde auf Blechen verteilt und eine und 90 Min. bei 175°C im Umlufttrockenschrank getrocknet. Die Beladung der Bleche mit Polymergel betrug 0,81 g/cm$^2$.

Das getrocknete Polymergel wurde mittels eines einstufigen Walzenstuhles gemahlen (drei Mahlgänge, 1. Mahlgang mit Spaltbreite 1000μm, 2. Mahlgang mit Spaltbreite 600μm und 3. Mahlgang mit Spaltbreite 400μm). Das gemahlene getrocknete Polymergel wurde klassiert und eine synthetische Partikelgrößenverteilung (PSD) mit folgender Zusammensetzung abgemischt:

600 bis 710μm: 10,6 Gew.%
500 bis 600μm: 27,9 Gew.%
300 bis 500μm: 42,7 Gew.%
200 bis 300μm: 13,8 Gew.%
150 bis 200μm: 5,0 Gew.%

Das so erhaltene Grundpolymer E wurde analysiert. Die Ergebnisse sind in Tabelle 3 eingetragen.

Beispiel 10 (Vergleichsbeispiel)

Das Grundpolymer E aus Beispiel 9 wurde wie in Beispiel 2 thermisch oberflächennachvernetzt. Die Fraktion mit einer Partikelgröße von kleiner 710 μm wurde analysiert. Die Ergebnisse sind in Tabelle 4 eingetragen.

Beispiel 11

Es wurde verfahren wie unter Beispiel 9, wobei die Einsatzmenge des Vernetzers, bezogen auf die eingesetzte Acrylsäure, 0,28 Gew.-% betrug. Das so erhaltene Grundpolymer F wurde analysiert. Die Ergebnisse sind in Tabelle 3 eingetragen.

Beispiel 12

Das Grundpolymer F aus Beispiel 11 wurde wie in Beispiel 2 thermisch oberflächennachvernetzt. Die Fraktion mit einer Partikelgröße von kleiner 710 μm wurde analysiert. Die Ergebnisse sind in Tabelle 4 eingetragen.

Beispiel 13

Es wurde verfahren wie unter Beispiel 9, wobei die Einsatzmenge des Vernetzers, bezogen auf die eingesetzte Acrylsäure, 0,35 Gew.-% betrug. Das so erhaltene Grundpolymer G wurde analysiert. Die Ergebnisse sind in Tabelle 3 eingetragen.

Beispiel 14

Das Grundpolymer G aus Beispiel 13 wurde wie in Beispiel 2 thermisch oberflächennachvernetzt. Die Fraktion mit einer Partikelgröße von kleiner 710 μm wurde analysiert. Die Ergebnisse sind in Tabelle 4 eingetragen.

Beispiel 15

Es wurde verfahren wie unter Beispiel 1, wobei die Einsatzmenge des Vernetzers, bezogen auf die eingesetzte Acrylsäure, 0,43 Gew.-% betrug. Die Temperatur des Polymergels bei der Extrusion betrug 85°. Das so erhaltene Grundpolymer H wurde analysiert. Die Ergebnisse sind in Tabelle 3 eingetragen.

Beispiel 16

Das Grundpolymer H aus Beispiel 15 wurde wie in Beispiel 2 thermisch oberflächennachvernetzt. Die Fraktion mit einer Partikelgröße von kleiner 710 μm wurde analysiert. Die Ergebnisse sind in Tabelle 4 eingetragen.

Tab. 3: Vernetzereinfluss auf das Grundpolymer

| Bsp. | Vernetzer [Gew.-%] | CRC [g/g] | AUL0.3psi [g/g] | FSR [g/gs] |
|------|--------------------|-----------|-----------------|------------|
| 9*)  | 0,20               | 37,3      | 8,8             | 0,31       |
| 11   | 0,28               | 34,3      | 11,8            | 0,35       |
| 13   | 0,35               | 33,5      | 15,1            | 0,35       |
| 15   | 0,43               | 32,5      | 22,9            | 0,40       |
| *) Vergleichsbeispiele | | | | |

Tab. 4: Vernetzereinfluss auf das Endprodukt

| Bsp. | Vernetzer [Gew.-%] | SFC[$10^{-7}$ x cm$^3$s/g] | CRC [g/g] | AUL0.7psi [g/g] | FSR [g/gs] |
|---|---|---|---|---|---|
| 10*) | 0,20 | 106 | 25,8 | 23,2 | 0,24 |
| 12 | 0,28 | 121 | 26,4 | 25,0 | 0,26 |
| 14 | 0,35 | 135 | 26,3 | 23,9 | 0,29 |
| 16 | 0,43 | 122 | 26,4 | 24,6 | 0,32 |
| *) Vergleichsbeispiele | | | | | |

Die Beispiele 9 bis 16 zeigen, dass die Quellgeschwindigkeit (FSR) nach der Oberflächennachvernetzung mit der verwendeten Vernetzermenge in den extrudierten Grundpolymeren steigt.

Beispiel 17 (Vergleichsbeispiel)

Es wurde verfahren wie unter Beispiel 9, wobei das erhaltene Polymergel nicht extrudiert wurde. Das so erhaltene Grundpolymer I wurde analysiert. Die Ergebnisse sind in Tabelle 5 eingetragen.

Beispiel 18 (Vergleichsbeispiel)

Das Grundpolymer I aus Beispiel 17 wurde wie in Beispiel 2 thermisch oberflächennachvernetzt. Die Fraktion mit einer Partikelgröße von kleiner 710 $\mu$m wurde analysiert. Die Ergebnisse sind in Tabelle 6 eingetragen.

Beispiel 19 (Vergleichsbeispiel)

Es wurde verfahren wie unter Beispiel 11, wobei das erhaltene Polymergel nicht extrudiert wurde. Das so erhaltene Grundpolymer J wurde analysiert. Die Ergebnisse sind in Tabelle 5 eingetragen.

Beispiel 20 (Vergleichsbeispiel)

Das Grundpolymer J aus Beispiel 19 wurde wie in Beispiel 2 thermisch oberflächennachvernetzt. Die Fraktion mit einer Partikelgröße von kleiner 710 $\mu$m wurde analysiert. Die Ergebnisse sind in Tabelle 6 eingetragen.

Beispiel 21 (Vergleichsbeispiel)

Es wurde verfahren wie unter Beispiel 13, wobei das erhaltene Polymergel nicht extrudiert wurde. Das so erhaltene Grundpolymer K wurde analysiert. Die Ergebnisse sind in Tabelle 5 eingetragen.

Beispiel 22 (Vergleichsbeispiel)

Das Grundpolymer K aus Beispiel 21 wurde wie in Beispiel 2 thermisch oberflächennachvernetzt. Die Fraktion mit einer Partikelgröße von kleiner 710 $\mu$m wurde analysiert. Die Ergebnisse sind in Tabelle 6 eingetragen.

Beispiel 23 (Vergleichsbeispiel)

Es wurde verfahren wie unter Beispiel 15, wobei das erhaltene Polymergel nicht extrudiert wurde. Das so erhaltene Grundpolymer L wurde analysiert. Die Ergebnisse sind in Tabelle 5 eingetragen.

Beispiel 24 (Vergleichsbeispiel)

Das Grundpolymer L aus Beispiel 23 wurde wie in Beispiel 2 thermisch oberflächennachvernetzt. Die Fraktion mit einer Partikelgröße von kleiner 710 $\mu$m wurde analysiert. Die Ergebnisse sind in Tabelle 6 eingetragen.

Tab. 5: Vernetzereinfluss auf das Grundpolymer

| Bsp. | Vernetzer [Gew.-%] | CRC [g/g] | AUL0.3psi [g/g] | FSR [g/gs] |
|---|---|---|---|---|
| 17*) | 0,20 | 38,0 | 8,9 | 0,33 |
| 19*) | 0,28 | 34,9 | 11,7 | 0,32 |
| 21*) | 0,35 | 34,2 | 13,8 | 0,31 |
| 23*) | 0,43 | 34,2 | 17,3 | 0,31 |
| *) Vergleichsbeispiele | | | | |

Tab. 6: Vernetzereinfluss auf das Endprodukt

| Bsp. | Vernetzer [Gew.-%] | SFC [$10^{-7}$ x $cm^3$s/g] | CRC [g/g] | AUL0.7psi [g/g] | FSR [g/gs] |
|---|---|---|---|---|---|
| 18*) | 0,20 | 152 | 26,4 | 23,6 | 0,18 |
| 20*) | 0,28 | 160 | 26,1 | 23,9 | 0,18 |
| 22*) | 0,35 | 138 | 25,9 | 23,7 | 0,20 |
| 24*) | 0,43 | 107 | 26,4 | 23,7 | 0,18 |
| *) Vergleichsbeispiele | | | | | |

Die Beispiele 17 bis 24 zeigen, dass die verwendete Vernetzermenge in den Grundpolymeren bei fehlender Extrusion keinen signifikanten Einfluss auf die Quellgeschwindigkeit (FSR) nach der Oberflächennachvernetzung hat.

Beispiel 25 (Vergleichsbeispiel)

Es wurde verfahren wie unter Beispiel 15, wobei ein Teil des so erhaltenen Polymergels mit einem Extruder vom Typ OEE 8 (AMANDUS KAHL GmbH & Co. KG; Hamburg; Deutschland) extrudiert wurde. Die Temperatur des Polymergels bei der Extrusion betrug 85°. Die Lochplatte hatte 8 Löcher mit einem Lochdurchmesser von 8 mm. Die Dicke der Lochplatte betrug 15 mm.
Das Verhältnis von Innenlänge zu Innendurchmesser des Extruders (L/D) betrug 6,3. Die spezifische mechanische Energie (SME) der Extrusion betrug 89 kWh/t.
Das so erhaltene Grundpolymer M wurde analysiert. Die Ergebnisse sind in Tabelle 7 eingetragen.

Beispiel 26 (Vergleichsbeispiel)

Das Grundpolymer M aus Beispiel 25 wurde wie in Beispiel 2 thermisch oberflächennachvernetzt. Die Fraktion mit einer Partikelgröße von kleiner 710 $\mu$m wurde analysiert. Die Ergebnisse sind in Tabelle 8 eingetragen.

Tab. 7: Einfluss der SME auf das Grundpolymer

| Bsp. | SME [kWh/t] | CRC [g/g] | AUL0.3psi [g/g] | FSR [g/gs] |
|---|---|---|---|---|
| 15 | 26 | 32,5 | 22,9 | 0,40 |
| 25*) | 89 | 30,4 | 18,9 | 0,33 |
| *) Vergleichsbeispiel | | | | |

Tab. 8: Einfluss der SME auf das Endprodukt

| Bsp. | SME [kWh/t] | SFC [$10^{-7}$ x $cm^3$s/g] | CRC [g/g] | AUL0.7psi [g/g] | FSR [g/gs] |
|---|---|---|---|---|---|
| 16 | 26 | 122 | 26,4 | 24,6 | 0,32 |

(fortgesetzt)

| Bsp. | SME [kWh/t] | SFC [$10^{-7}$ x $cm^3$s/g] | CRC [g/g] | AUL0.7psi [g/g] | FSR [g/gs] |
|------|-------------|------------------------------|-----------|------------------|-------------|
| 26*) | 89 | 133 | 25,9 | 24,7 | 0,26 |
| *) Vergleichsbeispiel | | | | | |

Die Beispiele 16 und 26 zeigen, dass die Extrusion mit zu hoher spezifischer mechanischer Energie (SME) die Quellgeschwindigkeit (FSR) nach der Oberflächennachvernetzung senkt.

**Patentansprüche**

1. Verfahren zur Herstellung wasserabsorbierender Polymerpartikel durch Polymerisation einer Monomerlösung oder -suspension, enthaltend

   a) ein ethylenisch ungesättigtes, säuregruppentragendes Monomer, das zumindest teilweise neutralisiert sein kann,
   b) mindestens einen Vernetzer,
   c) mindestens einen Initiator,
   d) optional ein mit den unter a) genannten Monomeren copolymerisierbares ethylenisch ungesättigtes Monomer und
   e) optional ein oder mehrere wasserlösliche Polymere,

   in einem Polymerisationsreaktor mit mindestens zwei achsparallel rotierenden Wellen, Trocknung des erhaltenen Polymergels, Mahlung des getrockneten Polymergels, Klassierung und thermischer Oberflächennachvernetzung, **dadurch gekennzeichnet, dass** mindestens 0,25 Gew.-% des Vernetzers b), bezogen auf das unneutralisierte Monomer a), eingesetzt wird, dass das Polymergel vor der Trocknung extrudiert wird, dass das Polymergel während der Extrusion eine Temperatur größer 80°C aufweist und dass bei der Extrusion weniger als 60 kWh/t an spezifischer mechanischer Energie eingetragen wird.

2. Verfahren gemäß Anspruch 1, **dadurch gekennzeichnet, dass** das Polymergel während der Extrusion eine Temperatur von größer 90°C aufweist.

3. Verfahren gemäß Anspruch 1 oder 2, **dadurch gekennzeichnet, dass** der Extruder ein Verhältnis von Länge zu Durchmesser von weniger als 5 aufweist.

4. Verfahren gemäß einem der Ansprüche 1 bis 3, **dadurch gekennzeichnet, dass** das Öffnungsverhältnis der Lochplatte des Extruders im Bereich von 10 bis 20% liegt.

5. Verfahren gemäß einem der Ansprüche 1 bis 4, **dadurch gekennzeichnet, dass** der an der Lochplatte des Extruders anstehende Druck im Bereich von 15 bis 35 bar liegt.

6. Verfahren gemäß einem der Ansprüche 1 bis 5, **dadurch gekennzeichnet, dass** mindestens 0,4 Gew.-% des Vernetzers b), bezogen auf das unneutralisierte Monomer a), eingesetzt wird.

7. Verfahren gemäß einem der Ansprüche 1 bis 6, **dadurch gekennzeichnet, dass** durch Löcher mit einem Durchmesser von 8 bis 12 mm extrudiert wird.

8. Verfahren gemäß einem der Ansprüche 1 bis 7, **dadurch gekennzeichnet, dass** der Extruder begleitbeheizt wird.

9. Verfahren gemäß einem der Ansprüche 1 bis 8, **dadurch gekennzeichnet, dass** Monomer a) zu mindestens 50 mol-% teilweise neutralisierte Acrylsäure ist.

10. Verfahren gemäß einem der Ansprüche 1 bis 9, **dadurch gekennzeichnet, dass** das Monomer a) zu 25 bis 85 mol-% neutralisiert ist.

**EP 2 951 212 B1**

11. Wasserabsorbierende Polymerpartikel, erhältlich durch Polymerisation einer Monomerlösung oder -suspension, enthaltend

a) ein ethylenisch ungesättigtes, säuregruppentragendes Monomer, das zumindest teilweise neutralisiert sein kann,
b) mindestens einen Vernetzer,
c) mindestens einen Initiator,
d) optional ein mit den unter a) genannten Monomeren copolymerisierbares ethylenisch ungesättigtes Monomer und
e) optional ein oder mehrere wasserlösliche Polymere,

wobei mindestens 0,25 Gew.-% des Vernetzers b), bezogen auf das unneutralisierte Monomer a), eingesetzt wurde, und wobei das erhaltene Polymergel extrudiert wurde, die wasserabsorbierenden Polymerpartikel thermisch oberflächennachvernetzt wurden, die wasserabsorbierenden Polymerpartikel eine Quellgeschwindigkeit von mindestens 0,25 g/g s, eine Zentrifugenretentionskapazität von mindestens 25 g/g, eine Absorption unter einem Druck von 49,2 g/cm$^2$ von mindestens 24 g/g und eine Flüssigkeitsweiterleitung von mindestens 100 x 10$^{-7}$ cm$^3$s/g aufweisen, jeweils gemessen wie im Methodenteil der Beschreibung beschrieben.

12. Polymerpartikel gemäß Anspruch 11, wobei mindestens 0,4 Gew.-% des Vernetzers b), bezogen auf das unneutralisierte Monomer a), eingesetzt wurde.

13. Polymerpartikel gemäß Anspruch 11 oder 12, wobei die wasserabsorbierenden Polymerpartikel eine Quellgeschwindigkeit von mindestens 0,30 g/g s aufweisen.

14. Polymerpartikel gemäß einem der Ansprüche 11 bis 13, wobei die wasserabsorbierenden Polymerpartikel eine Flüssigkeitsweiterleitung von mindestens 110 x 10$^{-7}$ cm$^3$s/g aufweisen.

15. Hygieneartikel, enthaltend wasserabsorbierende Polymerpartikel gemäß einem der Ansprüche 11 bis 14.

**Claims**

1. A process for producing water-absorbing polymer particles by polymerizing a monomer solution or suspension comprising

a) an ethylenically unsaturated monomer which bears acid groups and may be at least partly neutralized,
b) at least one crosslinker,
c) at least one initiator,
d) optionally an ethylenically unsaturated monomer copolymerizable with the monomers mentioned under a) and
e) optionally one or more water-soluble polymers,

in a polymerization reactor having at least two shafts rotating in an axially parallel manner, drying the resulting polymer gel, grinding the dried polymer gel, classifying and thermally surface postcrosslinking, which process comprises using at least 0.25% by weight of the crosslinker b), based on the unneutralized monomer a), extruding the polymer gel prior to drying, the polymer gel during the extrusion having a temperature greater than 80°C and less than 60 kWh/t of specific mechanical energy being introduced in the course of extrusion.

2. The process according to claim 1, wherein the polymer gel during the extrusion has a temperature of greater than 90°C.

3. The process according to claim 1 or 2, wherein the extruder has a ratio of length to diameter of less than 5.

4. The process according to any of claims 1 to 3, wherein the orifice ratio of the perforated plate of the extruder is in the range from 10 to 20%.

5. The process according to any of claims 1 to 4, wherein the pressure bearing on the perforated plate of the extruder is in the range from 15 to 35 bar.

**6.** The process according to any of claims 1 to 5, wherein at least 0.4% by weight of the crosslinker b), based on the unneutralized monomer a), is used.

**7.** The process according to any of claims 1 to 6, wherein extrusion is effected through holes having a diameter of 8 to 12 mm.

**8.** The process according to any of claims 1 to 7, wherein the extruder is trace-heated.

**9.** The process according to any of claims 1 to 8, wherein at least 50 mol% of monomer a) is partly neutralized acrylic acid.

**10.** The process according to any of claims 1 to 9, wherein monomer a) has been neutralized to an extent of 25 to 85 mol%.

**11.** Water-absorbing polymer particles obtainable by polymerizing a monomer solution or suspension comprising

  a) an ethylenically unsaturated monomer which bears acid groups and may be at least partly neutralized,
  b) at least one crosslinker,
  c) at least one initiator,
  d) optionally an ethylenically unsaturated monomer copolymerizable with the monomers mentioned under a) and
  e) optionally one or more water-soluble polymers,

wherein at least 0.25% by weight of the crosslinker b), based on the unneutralized monomer a), has been used, and wherein the resulting polymer gel has been extruded, the water-absorbing polymer particles have been thermally surface postcrosslinked, the water-absorbing polymer particles have a free swell rate of at least 0.25 g/g s, a centrifuge retention capacity of at least 25 g/g, an absorption under a pressure of 49.2 g/cm$^2$ of at least 24 g/g and a saline flow conductivity of at least 100 x 10$^{-7}$ cm$^3$s/g, each measured as described in the method section of the description.

**12.** Polymer particles according to claim 11, wherein at least 0.4% by weight of the crosslinker b), based on the unneutralized monomer a), has been used.

**13.** Polymer particles according to claim 11 or 12, wherein the water-absorbing polymer particles have a free swell rate of at least 0.30 g/g s.

**14.** Polymer particles according to any of claims 11 to 13, wherein the water-absorbing polymer particles have a saline flow conductivity of at least 110 x 10$^{-7}$ cm$^3$s/g.

**15.** A hygiene article comprising water-absorbing polymer particles according to any of claims 11 to 14.


**Revendications**

**1.** Procédé pour la préparation de particules polymères absorbant l'eau par polymérisation d'une solution ou d'une suspension de monomères, contenant

  a) un monomère éthyléniquement insaturé, portant des groupes acides, qui peut être au moins partiellement neutralisé,
  b) au moins un réticulant,
  c) au moins un initiateur,
  d) éventuellement un monomère éthyléniquement insaturé copolymérisable avec les monomères cités en a) et
  e) éventuellement un ou plusieurs polymères solubles dans l'eau,

dans un réacteur de polymérisation présentant au moins deux arbres rotatifs à axes parallèles, séchage du gel polymère obtenu, broyage du gel polymère séché, classification et postréticulation thermique en surface, **caractérisé en ce qu'**on utilise au moins 0,25% en poids du réticulant b) par rapport au monomère a) non neutralisé, **en ce que** le gel polymère est extrudé avant le séchage, **en ce que** le gel polymère présente une température supérieure à 80°C pendant l'extrusion et **en ce que** moins de 60 kWh/t d'énergie mécanique spécifique sont introduits lors de l'extrusion.

**2.** Procédé selon la revendication 1, **caractérisé en ce que** le gel polymère présente une température supérieure à 90°C pendant l'extrusion.

**3.** Procédé selon la revendication 1 ou 2, **caractérisé en ce que** l'extrudeuse présente un rapport de longueur à diamètre inférieur à 5.

**4.** Procédé selon l'une quelconque des revendications 1 à 3, **caractérisé en ce que** le rapport d'ouverture de la plaque perforée de l'extrudeuse se situe dans la plage de 10 à 20%.

**5.** Procédé selon l'une quelconque des revendications 1 à 4, **caractérisé en ce que** la pression qui s'exerce sur la plaque perforée de l'extrudeuse se situe dans la plage de 15 à 35 bars.

**6.** Procédé selon l'une quelconque des revendications 1 à 5, **caractérisé en ce qu'**on utilise au moins 0,4% en poids du réticulant b), par rapport au monomère a) non neutralisé.

**7.** Procédé selon l'une quelconque des revendications 1 à 6, **caractérisé en ce qu'**on extrude au travers de trous présentant un diamètre de 8 à 12 mm.

**8.** Procédé selon l'une quelconque des revendications 1 à 7, **caractérisé en ce que** l'extrudeuse est chauffée de manière auxiliaire.

**9.** Procédé selon l'une quelconque des revendications 1 à 8, **caractérisé en ce que** le monomère

   a) est, à raison d'au moins 50% en mole, de l'acide acrylique partiellement neutralisé.

**10.** Procédé selon l'une quelconque des revendications 1 à 9, **caractérisé en ce que** le monomère

   a) est neutralisé à raison de 25 à 85% en mole.

**11.** Particules polymères absorbant l'eau, pouvant être obtenues par polymérisation d'une solution ou d'une suspension de monomères, contenant

   a) un monomère éthyléniquement insaturé, portant des groupes acides, qui peut être au moins partiellement neutralisé,
   b) au moins un réticulant,
   c) au moins un initiateur,
   d) éventuellement un monomère éthyléniquement insaturé copolymérisable avec les monomères cités en a) et
   e) éventuellement un ou plusieurs polymères solubles dans l'eau,

au moins 0,25% en poids du réticulant b) par rapport au monomère a) non neutralisé ayant été utilisé et le gel polymère obtenu ayant été extrudé, les particules polymères absorbant l'eau ayant été postréticulées thermiquement en surface, les particules polymères absorbant l'eau présentant une vitesse de gonflement d'au moins 0,25 g/g.s, une capacité de rétention à la centrifugeuse d'au moins 25 g/g, une absorption sous une pression de 49,2 g/cm$^2$ d'au moins 24 g/g et une transmission des liquides d'au moins 100 x 10$^{-7}$ cm$^3$s/g, à chaque fois mesurée comme décrit dans la partie procédé de la description.

**12.** Particules polymères selon la revendication 11, au moins 0,4% en poids du réticulant b), par rapport au monomère a) non neutralisé étant utilisé.

**13.** Particules polymères selon la revendication 11 à 12, les particules polymères absorbant l'eau présentant vitesse de gonflement d'au moins 0,30 g/g.s.

**14.** Particules polymères selon l'une quelconque des revendications 11 à 13, les particules polymères absorbant l'eau présentant une transmission des liquides d'au moins 110 x 10$^{-7}$ cm$^3$s/g.

**15.** Objets hygiéniques contenant des particules polymères absorbant l'eau selon l'une quelconque des revendications 11 à 14.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 01038402 A1
- WO 03022896 A1
- WO 03051415 A1
- WO 2006034806 A1
- WO 2006034853 A1
- WO 2009115472 A1
- EP 0497623 A2
- WO 2005097313 A1
- WO 2012107432 A
- WO 2012160174 A
- WO 2013007819 A
- CH 664704 A5
- EP 0517068 A1
- WO 9712666 A1
- DE 2123956 A1
- EP 0603525 A1
- DE 19536944 A1
- DE 4118884 A1
- WO 02055469 A1
- WO 03078378 A1
- WO 2004035514 A1
- EP 0530438 A1
- EP 0547847 A1
- EP 0559476 A1
- EP 0632068 A1
- WO 9321237 A1
- WO 03104299 A1
- WO 03104300 A1
- WO 03104301 A1
- DE 10331450 A1
- DE 10331456 A1
- DE 10355401 A1
- DE 19543368 A1
- DE 19646484 A1
- WO 9015830 A1
- WO 02032962 A2
- EP 0083022 A2
- EP 0543303 A1
- EP 0937736 A2
- DE 3314019 A1
- DE 3523617 A1
- EP 0450922 A2
- DE 10204938 A1
- US 6239230 B
- DE 4020780 C1
- DE 19807502 A1
- DE 19807992 C1
- DE 19854573 A1
- DE 19854574 A1
- DE 10204937 A1
- DE 10334584 A1
- EP 1199327 A2
- WO 03031482 A1
- DE 3713601 A1
- EP 0640330 A1

**In der Beschreibung aufgeführte Nicht-Patentliteratur**

- **F.L. BUCHHOLZ ; A.T. GRAHAM.** Modern Superabsorbent Polymer Technology. Wiley-VCH, 1998, 71-103
- Standard Test Methods for the Nonwovens Industry. 2005